# EUROPEAN PATENT SPECIFICATION

(11) **EP 4 059 022 B1**
(45) Date of publication and mention of the grant of the patent: **16.07.2025**
(21) Application number: 20797137.5
(22) Date of filing: 02.11.2020
(51) Int. Cl.: G16B 20/00, A01M 1/02, A01M 21/00, A01B 79/00

(54) **CROP MONITORING AND PROTECTION**
ERNTEGUTÜBERWACHUNG UND -SCHUTZ
SURVEILLANCE ET PROTECTION DE RÉCOLTES

(30) Priority: 13.11.2019 EP 19208777; 15.05.2020 EP 20174872
(43) Date of publication of application: 21.09.2022
(73) Proprietor: BASF SE, 67056 Ludwigshafen am Rhein (DE)
(72) Inventor: TRESCH, Stefan, 67117 Limburgerhof (DE); SIEPE, Isabella, 67117 Limburgerhof (DE); BUSCH, Kristina, 67117 Limburgerhof (DE); STAMMLER, Gerd, 67117 Limburgerhof (DE); MONTAG, Jurith, 67117 Limburgerhof (DE)
(74) Representative: BASF IP Association
(86) International application number: PCT/EP2020/080623
(87) International publication number: WO 2021/094115

(56) References cited:
- EP-A1- 3 287 007
- WO-A1-2015/132208
- CA-A1- 2 969 282

## Description

### Technical field

The present teachings relate generally to computer assisted identification of an organism influencing a crop. The present teachings also relate to computer assisted management and control of one or more of such organisms.

### Background art

Harmful organisms such as weeds, and plant pests, such as parasitic bacteria, fungi, insects, mites, nematodes or viruses and viroids can cause significant damage to a crop. Chemical compounds or biologicals such as herbicides, fungicides, insecticides, miticides or nematicides are used in agriculture to control such weeds and plant pests and are commonly known as pesticides.

It is known that some harmful organisms may respond to pesticidal chemical or biological compounds by evolving and developing resistance against the compounds to which these organisms have been exposed. The organism may, for example, mutate and produce a variety which has a high degree of tolerance or are even resistance to the applied pesticide compound. The organism may even become resistant to other pesticidal chemical compounds; a phenomenon which is commonly known as cross-resistance. Such an applied pesticide compound may thus become ineffective against the resistant organism. Accordingly, application of such a compound, and other compounds to which cross-resistance exists, to control the resistant organism may be ecologically and financially undesirable. As mentioned, the organism may become resistant, for example by mutation or by shifting.

In WO19149626 a control of resistant harmful organisms is proposed, which is based on DNA/RNA sequencing technique.

CA2969282A1 discloses a risk assessment methodology taking into account an analysis of collected spores and weather data. In an example, spores are first identified by optical microscopy, which is optionally supplemented by PCR analysis, wherein the primers are chosen. This prior art does not disclose in-situ genotyping methods, and does not select a suitable sensor arrangement for genotyping based on a combination of crop data and region data.

There is thus a requirement for a cost-effective method for on-site identification of an organism.

### Summary

At least some of the problems inherent to the prior-art will be shown solved by the subject-matter of the accompanying independent claims.

When viewed from a first perspective of the present teachings there can be provided a computer-implemented method comprising:
- receiving, at a processing means, a crop data indicative of the type of a crop;
- receiving, at the processing means, a region data indicative of at least one harmful organism that may be present at or around the location of the crop;
- selecting from a database, using the processing means, a sensor arrangement suitable for selectively genotyping at least one relevant harmful organism; wherein the at least one relevant harmful organism is among the at least one harmful organism, and wherein the selection is performed in response to the crop data and the region data.

Herein, the sensor arrangement is suitable for in-situ genotyping the at least one relevant harmful organism, wherein the sensor arrangement is a lab-on-a-chip device.

The embodiments and preferred embodiments described for the computer-implemented method are also valid for other objects of the invention, in particular the electronic device and the computer program. It will be understood that even though these are not reiterated for each and every aspect of the present invention, they are nevertheless applicable mutatis mutandis.

The term "resistant" and mutatis mutandis "resistance", as used in "pesticide resistant pest", "fungicide resistant" relates to a property of the thus characterized organism to have a reduced susceptibility to the treatment with a pesticide. Susceptibility may be measured by the desired effect that the pesticide treatment aims for, e.g. growth regulation, prevention of infestation, pre-emergence control, control of present harmful organisms etc. Typically, the susceptibility will be measured by mortality of the harmful organism upon treatment with a pesticide as compared to a non-treated control of the harmful organism. If treatment is carried out pre-emergence (e.g. on a crop plant, or to the locus, such as the soil where the plant is growing or will be grown), susceptibility may also be measured by ratio of emerged harmful organisms as compared to a non-treated control. When referring to "resistant pests" or "resistances" in the present text, the susceptibility of the pesticide resistant harmful organism will be at least 10% lower than the control group, preferably at least 20 % lower, more preferably at least 50 % lower, and in particular at least 80 % lower than the control.

The terms "in-situ" and "on-site" are equivalent and relate the situation that the genotyping measurement is performed essentially at the locus where the sampled material for measurement is collected and/or prepared. The same locus comprises both the immediate vicinity to the place where the sampling was carried out (e.g. within a radius of 1000 meters, such as within a radius of 100 meters), but may also relate to the functional or organizational unit where the sampling was carried out, such as a farm, a breeding station, a laboratory, a greenhouse etc. The terms "in-situ" and "on-site" thus exclude any techniques that require the shipping of a sample to a specialized analytical facility, such as sequencing technologies. Accordingly, the terms "in-situ" and "on-site" refer to a situation wherein the method steps from the sampling to the generation of the result signals or the processed result signal is carried out over a period of up to 24 hours, preferably up to 3 hours, more preferably up to 1 hour, and especially preferably up to 30 minutes.

The term "around", as used in "around the location of the crop" both relates to the immediate vicinity to the place where the sampling was carried out (e.g. within a radius of 1000 meters, such as within a radius of 100 meters), but may also relate to the functional or organizational unit where the sampling was carried out, such as a farm, neighboring farms, a breeding station, a laboratory, a greenhouse etc, and even to the regional unit of the locus of the crop plant and neighboring regional units sharing the same or comparable environmental conditions, e.g. weather including precipitation, solar irradiation intensity, soil type, height above sea level, median temperature etc., Typically, the term "around" may relate to a radius of up to 50 km, preferably up to 10 km from the locus where the crop plant is growing or is intended to grow.

The term "locus" as used in "the locus of the crop (plant)" relates to the soil, area, material or environment in which a crop plant is growing or may grow.

The terms "active" and "present" in the context of a harmful organism are equivalent. It will be appreciated that the choice of term will be dependent on the type of harmful organism. For example, undesired vegetation such as weeds would usually be described to be present, whereas mobile animals like insects would be described as active.

The term "relevant" as used in the context of "relevant harmful organism(s)" relates to a subgroup of harmful organisms, more precisely to those harmful organisms that are both not unlikely to occur as signified by the region data, and which also negatively affect the particular crop of interest.

The terms "via the processing means" and "using the processing means" are equivalent and relate to a situation, wherein an action is performed automatically by the processing means i.e. without any further user interaction with the processing means.

The applicant has realized that by combining the crop data with the region data, the scope of detection for the harmful organisms that can be present on the location or on site can be significantly narrowed to focus on one or more relevant organisms. Furthermore, genotyping techniques may be applied subsequently to this narrowed scope of interest to gain further advantages as will be outlined below.

It will be understood that a crop is a product, typically plant, that can be grown and harvested for profit or subsistence. The crop may be cultivated in agriculture or in aquaculture. The crop may be cultivated indoors or outdoors. The crop may be a food crop, such as food grains, feed crop, edible seeds, fruits or vegetables, or it may even be a non-food crop, such as floriculture, turf or an industrial crop (e.g., biofuel, fiber, etc.).

Even on an individual level of each of the two data, the scope can be limited according to relevance. For example, the crop data, which comprises information related to the crop, such as the type of crop can be used to limit the scope of detection to the sub-set of harmful organisms that do affect the crop of interest. As a further example, if the crop type is wheat, then the harmful organisms that only affect potatoes can be excluded from the scope of testing. It will be appreciated that certain harmful organisms may only affect certain variety or sub-type of the crop. For example, a certain organism may affect wheat species "*Spelt*", but not another wheat species "*Durum*". In such case, the crop data may also comprise information related to the specific species or even the variety of the species for further narrowing down the scope of testing.

It will be appreciated that the crop data may pertain to a crop that is already present on the site, or it may even pertain to a crop that is being planned to be grown on the site.

Similarly, the region data provides information related to one or more harmful organisms that may be present at or around the location of the crop. In one embodiment, the region data contains information about the occurrence of a relevant harmful organism or a part thereof (such as spores) at or around the location of the crop. In another embodiment, the region data contains information about the occurrence of a relevant harmful organism or a part thereof (such as spores) around the location of the crop. For example, if incidents of *soybean rust* and *powdery mildew* have been detected within a certain distance of the crop, these two types of harmful organisms may be used to define the scope of the test. By certain distance it is meant say within the same region, for example, 1 km from the crop. Exact distance or definition of the region is not limiting to the scope or generality of the present teachings as it depends on the likelihood or certainty with which the test results are desired. For example, if there is a reported incident of *powdery mildew* within 50 m of the crop it is more likely that the same harmful organism, i.e., *powdery mildew,* is also affecting at least some portion of the crop, as compared to the case where the harmful organism is detected, say, 1 km away from the crop.

By saying here that the region data provides information related to one or more harmful organisms that may be active or present at or around the location of the crop, it is also meant that region data may also comprise information on resistance genes of the one or more harmful organisms that may be active or present at or around the location of the crop. For example, the region data may provide information on the presence of a pesticide resistant harmful organism at or around the location of the crop, preferably also to which pesticide the harmful organism is resistant, more preferably also the genes and/or mutations responsible for said resistance. Such information may also relate to previous growing seasons, i.e. the region data may contain information on the presence of the pesticide resistant harmful organism during a previous growing season at or around the location of the crop, preferably also to which pesticide the harmful organism is resistant, more preferably also the genes and/or mutations responsible for said resistance.

In one embodiment, the region data provides information related to the type of pesticide resistance of a harmful organism that is present at or around the location of the crop. In another embodiment, the region data provides information related to the type of pesticide resistance of a harmful organism that was present at or around the location of the crop during a previous growing season.

The region data may even comprise information on past physical and/or chemical treatments of a crop or the field where the crop is growing or is intended to grow. Physical treatments may for example relate to the application of agronomic machinery, burning of weeds, drowning of weeds etc. Chemical treatments may relate to the application and dosage of previous treatments with agrochemicals, such as fungicides, insecticides, or herbicides. In one embodiment, the region data comprises information on pesticide application such as utilized pesticide and application rates in previous growing seasons, preferably in previous and the current growing season. It will be appreciated that resistant harmful organisms are more likely to occur in areas, which favor the biological selection of resistant populations to the harmful organism. Thus, information on previous chemical treatments may be used to assess the risk of pesticide resistances at or around the location where a crop is growing or is intended to grow.

If the region data includes information on the occurrence of a relevant harmful organism, the inventive method is typically applied for detecting a DNA-sequence that is indicative of a pesticide resistance, e.g. a fungicide resistance, as described herein below. A DNA-sequence is indicative of a pesticide resistance if the DNA-sequence either functionally conveys the pesticide resistance, e.g. by giving the harmful organism to ability to degrade a chemical, or it does not functionally interfere with the interaction of a pesticide with the organism, but is a marker for other DNA-sequences that in turn do functionally interfere with the interaction of the pesticide with said organism. Accordingly, the method may be applied for detecting a DNA-sequence that is indicative of a pesticide resistance, and wherein the sensor arrangement is configured to detect the DNA-sequence that is indicative of the pesticide resistance.

Hence, by combining the crop data with the region data, the scope of testing is synergistically improved by mutually excluding the crop types and harmful organisms respectively that are not relevant to the other of the two data. Accordingly, the processing means is thus able to use the crop data and the region data to automatically find, from a database, the requirements for a sensor arrangement that is best suited for selectively detecting the presence of one or more of the relevant harmful organisms for the crop. It will be understood that selectively detecting means specifically detecting, or testing for the presence of, a given relevant harmful organism.

It will be understood that by saying "the at least one relevant harmful organism is among the at least one harmful organism", it is meant that the at least one harmful organism is also indicated by the region data. Accordingly, each relevant harmful organism is that which is relevant for the crop and is also indicated by the region data.

It will further be understood that the selection is performed in response to the crop data and the region data such that their combination indicates the at least one relevant harmful organism. According to an aspect, the crop data is indicative of the harmful organisms that can affect the crop. This can speed up the determination the scope of testing, i.e., determining which of the harmful organisms are relevant for detection, by defining an organism as a relevant harmful organism if the harmful organism is also indicated in the region data. Alternatively, the processing means can determine the scope of testing by combining the crop data with the region data and determining the scope of detection by accessing additional data from the database. The processing means can thus select the sensor arrangement suitable for selectively genotyping the at least one relevant harmful organism from the database. The additional data may for example be data related to the harmful organisms that can affect the crop.

In one embodiment, the region data indicative of at least one harmful organism does not comprise information on the occurrence of the at least one (relevant) harmful organism or parts thereof (such as spores) that may be present at or around the location of the crop, especially no data that are based on analytic measurements or observations performed on the at least one (relevant) harmful organism at or around the location of the crop, such as data retrieved from visual identification means (e.g. microscopic detection) or genetic identification means (e.g. PCR-based techniques or sequencing techniques). In another embodiment, the region data does not contain any information on the identity of the at least one (relevant) harmful organism that may be present at or around the location of the crop, preferably no information on the genetic identity of the at least one (relevant) harmful organism.

According to another aspect, the region data comprises season data. The season data comprises information about the time of the year, which can be used by the processing means to further narrow down number of the harmful organisms that can be of interest for the crop.

According to another aspect, the region data may comprise weather data, which can be used by the processing means to further determine which of the harmful organisms can be more relevant given the conditions that are prevailing for the crop. Those skilled in the art with appreciate that prevailing conditions such as wind intensity, wind direction, humidity and ambient temperature are further factors that can determine the likelihood with respect to the distance of the crop from the previously reported incident of the harmful organism. The prevailing conditions may be one or more of: temperature, humidity, wind speed, wind direction, and precipitation, either at a given time and/or within a predetermined time period.

Season and/or weather data can thus further be used to preselect/select the at least one relevant harmful organism or at least one harmful organism of interest.

According to yet another aspect, the region data comprises a location data relating to a geographical position of the crop. The processing means may thus determine the geographical position of the crop using the region data.

According to an aspect, the processing means automatically retrieves, from one or more databases, any of the: information related to one or more harmful organisms that may be active or present at or around the location of the crop, season data, and weather data in response to determining the geographical position of the crop.

Such further data, e.g., weather data and/or season data, and/or location data, within the region data can thus further allow the processing means to narrow the scope of testing by further excluding the harmful organisms that can affect to the crop but are not relevant for testing given the further data.

In one embodiment, the region data is selected from season data, weather data, and location data. In another embodiment, the region data comprises season data. In another embodiment, the region data comprises weather data. In another embodiment, the season data comprises location data.

The sensor arrangement can either be a single device that can selectively detect each of the relevant organisms to be tested, or it can also be a plurality of devices or sensors each of which is individually designed for selectively detecting a specific organism. Each sensor may comprise an assay which is specific for detecting a specific organism. In this context, an organism with at least one single nucleotide polymorphism is considered a different organism. For example, the sensor arrangement may be a single lab-on-chip device that has specific assays or capability to detect each of the relevant organisms. Alternatively, the sensor arrangement may even be a plurality of different lab-on-chip devices each of which is capable of detecting a single specific organism different from the other devices. The sensor arrangement may even be a combination of a lab-on-chip device that is capable of detecting multiple different organisms, and one or more lab-on-chip devices that are each capable of detecting a different specific organism.

The database may comprise data from a plurality of test results and field trials. For example, the database includes probe data for type, or even each specific mutation, of the harmful organism that has been measured in field trials and lab studies. The database may hence comprise selectivity data for each sensor, the selectivity data being indicative of the specific organism that each individual sensor can detect. The processing means may then select the appropriate sensor arrangement that is best suited to the requirements. In other words, the processing means selects the sensor arrangement that can reliably selectively detect each of the harmful organism that is determined to be relevant by the processing means.

As a further example, the selectivity data can be either one or more of: nucleic acid data, protein data or molecule data indicative of specific organisms. The sensor arrangement may comprise sensors based on nanopore technology. An advantage of nanopore technology can be the quantification capability. According to another aspect, the sensor arrangement may be at least partially based on microarray technology. An advantage of microarray-based arrangement is multiplexing capability. According to yet another aspect, the sensor arrangement may be at least partially based on a graphene biosensor. Graphene biosensors can possess higher sensitivity and/or selectivity, which can provide a sensor arrangement that is more selective and/or capable of detecting smaller traces of organisms (e.g. a CRISPR Cas modified graphene sensor system). High selectivity can be used for example for detecting more specific strains or mutations. According to another aspect or in addition to the above, the sensor arrangement is at least partially a microfluidics device. According to another aspect, the sensor arrangement may at least partially be a fast PCR system, for example based on a high-speed temperature cycling system or based on isothermal amplification (e.g. a loop-mediated-isothermal-amplification-type system). The sensor arrangement selected may even be a combination of the above technologies. The processing means can hence select the appropriate sensor arrangement, which is based on either one or more of these technologies, as best suited for the application at hand, determined by combining the crop data with the region data. According to another aspect, the sensor arrangement is selected based on the availability of the sensors. For example, if two or more different kind of sensors can selectively detect the same organism of interest, the processing means can select the sensor arrangement based on a given criterion. For example, the criteria that the sensor is selected can be based on which of the sensors is available or is most quickly available. According to another aspect, the selection criteria is, alternatively or in combination to above, including the cost of the sensor, or of the sensor arrangement that is to be used. Accordingly, the cheaper sensor can be preferred over an expensive sensor. Further alternatively or in combination to above, the selection criteria may include speed of sensing. Accordingly, a faster sensor may be selected over a slower one. It will be appreciated that by making available all or some of the above-mentioned criteria and data in the database, or in another database coupled to the database, the processing means can select a sensor arrangement that is optimized for detecting the at least one relevant harmful organism based upon given criteria and even on a given priority of the criteria.

The crop data may either be at least partially an input provided by a user, or it may be acquired automatically using a crop sensor, for example an image recognition system. The image recognition system comprises an image sensor such as a camera. By analyzing the image of the crop captured by the image sensor, the processing means may automatically determine the crop type. The image analysis may involve comparing data from the captured image with an image library or database. The crop data may even be acquired automatically by analyzing a biological sample from the crop, for example, by analyzing a part of the crop such as foliage, pollen, root, etc.

Similarly, the region data may either be at least partially be an input provided by a user. Alternatively, the region data is at least partially retrieved from a region database or a remote server. For example, after determining the location of the crop, either via a user input or automatically via a geolocation means (such as a GPS, and/or a beacon, etc.) the processing means may automatically retrieve from a public database information about which harmful organisms may be present at or around the location of the crop. According to an aspect, the region data also comprises data from previous measurements of harmful organisms performed at or around the location of the crop, e.g. detections or measurements without detections from previous growing seasons.

Different types of organisms can influence crops in different ways. If an organism affects a crop, i.e., has a negative influence on a crop, such an organism can be called a harmful organism. Alternatively, if an organism has a positive effect on a crop, the organism can be called a benign organism.

It will be understood that the harmful organism may be any of the: infectious microorganism or microbes or macrobials such as fungi, bacteria, viruses, viroids, oomycetes, nematodes, proto-zoa, phytoplasma, insects and their likes that affect a crop. The term harmful organism may even encompass pests as well as weeds that can affect crop health at any stage of cultivation. In present disclosure the terms harmful organism and pathogen will be used interchangeably, henceforth the term pathogen is also implied to encompass weeds and pests that affect a crop. The harmful organism may either affect the crop directly, or it may do so indirectly. For example, ants are known to have a mutualistic relationship with aphids. So, even though ants may not attack a crop directly, but by tending to the aphids, which do cause damage to the crop, ants may be considered a harmful organism to that crop. Typically, the term "harmful organism" relates to unwanted vegetation (e.g. weeds), fungi, arthropods (e.g. insects and arachnids), mollusks (e.g. snails), nematodes, viruses, and bacteria, preferably fungi (e.g. Septoria sp.).

It has been further realized that the proposed methods can also be extended to detection of one or more benign organisms. It will be appreciated that a benign organism may either have a positive direct influence on the crop, or an indirect one. In other words, it is possible that a benign organism does not provide a direct effect on the crop, but an indirect one. For example, if a first organism does not directly influence a crop, but it affects, or has a negative influence on, a second organism, and if the second organism is a harmful organism for the crop, then the first organism may still be considered a benign organism for that crop.

Some consumers may prefer use of a biological alternative to a chemical active ingredient. For example, in organic farming the use of naturally occurring substances is desirable and use of synthetic substances is either avoided or strictly limited. Biological variants of chemical based pesticides, i.e., biopesticides may be more desirable in some markets. Such biological variants in many cases rely upon benign organisms such as benign- microbes or microorganisms. Biocontrol is another term which is used to refer to a method of controlling pests such as insects, mites, weeds and plant diseases using benign organisms. A treatment using such benign organisms may either be based on live organisms being applied to or around the crop, and/or an extract or parts of such benign organisms. For an effective biocontrol treatment, it may be beneficial to monitor the spread of the benign organism on or around the crop. As discussed previously, the benign organism may either be applied live and/or it may be a part or even extract of the benign organism that is applied.

Thus, according to an aspect, the sensor arrangement is selected by the processing means such that the arrangement is also suitable for selectively genotyping at least one benign organism.

The information related to the at least one benign organism may either be provided via the region data, or it may be provided as a separate input to the processing means. The processing means can thus select the sensor arrangement that can also detect the at least one benign organism. Similar to as it was outlined above, the detection of the benign organism may either be done via a single lab-on-chip device, or it may be done via different devices, each device specifically targeted towards a specific different organism, or it may be done by any combination of devices suitable for detecting the relevant organisms.

When viewed from another aspect, the method also comprises:
- outputting, via a human-machine interface functionally coupled to the processing means, information related to the suitable sensor arrangement.

The information on the suitable sensor arrangement is typically an indication which type of sensor arrangement should be used. It may also relate to an information on a combination of sensor arrangements that should be used.

Accordingly, the processing means can then output, via the human-machine-interface ("HMI") the information about the selected sensor arrangement. A user can thus be informed about the arrangement of a single or multiple specific lab-on-chip devices that should be used for testing. The human machine interface ("HMI") may comprise a video display unit (e.g., an LCD (Liquid Crystal Display), a CRT (Cathode Ray Tube) display, or a touch screen), an alphanumeric input device (e.g., a keyboard), a cursor control device (e.g., a mouse), and/or a signal generation device (e.g., a speaker). The HMI thus can, for example, be visual interface such as a screen and/or it can be an audio interface such as a loudspeaker. Accordingly, the output can either be displayed to the user and/or it can be announced via the speaker. Thus, the process of selecting and using the correct device for the detection of the relevant harmful organism can be made simple and intuitive for the user. For example, the devices can be intuitively coded, for example, using a color code. Accordingly, the HMI can output to the user that a "Blue" device should be used for the detection. The user can then pick a Blue device and use the Blue device for performing the detection. This can be particularly beneficial if several different kinds of devices are available for detecting different harmful organisms. Alternatively, or in addition, the devices may even be coded with an alphabetic, numeric, or alphanumeric code either of which is suitable for recognizing the correct device in a user-friendly manner. For example, the device may be called according to any of the respective schemes: device "1", device "A", or device "1B". According to an aspect, the device may be in the form of a cartridge that is used for a specific detection, or for a given plurality of organisms.

When the selected sensor arrangement is used, the measurement is selectively performed on the relevant organisms or even strains of interest that have been selected by combining the crop data with the region data. As a result, further significant time savings can be obtained as compared to sequencing the entire spectrum of strains. Thus, by combining: the selection of one or more relevant harmful organism strains, by combining the crop data and the region data; with a specifically chosen genotyping sensor that selectively tests for those relevant harmful organism strains, a combined benefit is achieved by the proposed method, which includes time and cost advantage.

Sequencing is typically a resource intensive activity as either the whole organism genome needs to be sequenced, which can produce large amounts of unnecessary data.

The applicant has realized that smaller areas of the genome that carry information for a pathogen or even a particular pesticide resistance can be specifically targeted for detection by combining the crop data and the region data. A particularly useful application of the present method is for detecting certain fungicide resistances of a fungal disease. The term "fungicide resistance" typically refers to a lower control rate of the fungal disease that can be achieved by application of a particular fungicide at a given concentration as compared with another (wild type) population of the fungal disease. Fungicide resistances are generated by various mechanisms, such as selection by fungicide treatment, natural or induced mutation, or sexual recombination. On a molecular level, fungicide resistance is often generated by an adaptation of the protein that is targeted by a fungicide, such as the exchange, deletion, and/or addition of (an) amino acid(s) in the primary sequence of the protein. These adaptations can be detected on the DNA level by a variety of techniques.

The inventive methods preferably utilize target-directed detection tools. Such target-directed detection tools are specific to a DNA sequence that is indicative of the pesticide resistance or pesticide tolerance, e.g. the fungicide resistance, such as a single nucleotide polymorphism ("SNP"). Ideally, target-directed detection tools are specific for exactly one DNA sequence. The result of target-directed detection tools is typically a classification of samples in "true" for samples containing the DNA-sequence looked for, and "wrong" for samples that do not contain the DNA-sequence searched for. They may also yield a quantitative result in that a value indicative of the amount of DNA-material containing the sequence to be detected is yielded. A combination of quantitative and qualitative information is also possible, such as a classified information based on pre-determined thresholds. In contrast to other DNA-analysis tools like sequencing, target-directed detection tools provide less information but are less expensive, less time-consuming and easier in their set-up as compared to unselective DNA analysis tools. Target-directed detection tools largely contribute to enabling an in-situ approach because they require far less equipment, less time-consuming, and still yield the required information.

Typical methods rely on hybridization of a probe oligonucleotide that has the sequence to be detected in a harmful organism (e.g. a fungus) - or its reverse complimentary sequence. The probe oligonucleotide may be RNA-based or DNA-based and is contacted with the DNA of the harmful organism, e.g. the fungus, to be analyzed. If the harmful organism has the corresponding DNA that is indicative of the pesticide resistance, a single strand of the fungal DNA will hybridize under suitable conditions with the probe oligonucleotide. The hybridization can be detected by various methods, such as polymerase-chain reaction technologies, fluorescence-based technologies, luminescence-based technologies, or electronic measurements (e.g. on a semiconductor chip such as a graphene chip).

Accordingly, the inventive method preferably contains the step of performing, in-situ, using the sensor arrangement connected at a sensor interface, genotyping of the at least one relevant harmful organism from an environmental sample obtained from a crop site, and generating a result signal via the sensor arrangement at the sensor interface, more preferably wherein the sensor arrangement includes a target-directed detection tool, most preferably a sequence-specific detection tool, especially preferably a sequence-specific detection tool that is based on the hybridization of a probe DNA- or RNA-molecule with the sequence to be identified, such as wherein the sequence to be identified is indicative of a pesticide resistance, e.g. a fungicide resistance. Accordingly, the sensor arrangement is typically suitable for in-situ genotyping the at least one relevant harmful organism.

A strength of the inventive method is also that it makes it possible to detect harmful organisms, e.g. fungal diseases, at a very early stage of the infestation of the plant, such as before the disease is detectable by visual assessment of material (e.g. fungal material like the spores), or the infected plant. This is particularly important for fungicide resistant fungal strains because these strains are hard to control and require specific measures by the applicant. By performing a preselection of a suitable sensor arrangement, taking into account crop data and region data, the inventive method may specifically test a sample on the presence of relevant harmful fungi. Since neither the crop data nor the region data rely on a visual detection of the fungal disease, the inventive method can even be applied at a point of time when there is still no visual information available. Accordingly, the inventive method may be performed before a harmful organism, such as a fungal disease, can be detected by visual assessment of the crop plant(s) to be analyzed.

Selection and use of such a sensor arrangement, for example in the form of one or more prefabricated sensors or lab-on-a-chip devices can be more user friendly.

Usually, the information about the possibility of presence of harmful organisms in an area is available in some format, for example, via public announcements by authorities or monitoring entities, internet database, etc. Such information may even be available from results from surrounding crops, or even from previous results from the same crop, which may even include sequencing results. Such information can be used to generate the region data. When this is combined with the crop data, the points of interest in the harmful organism spectrum can be significantly reduced such that measurements can be speed up to obtain quick results.

It is usually highly desirable to get the results as soon as possible, especially if a harmful organism is indeed present, for example, to start treatment immediately to prevent further damage to the crop, or even to start planning a treatment if the crop is yet to be grown. Even if visual information of the presence, or even outbreak of the harmful organism is available, it may not be sufficient to start a treatment as there is a risk that the strain of the organism is resistant to the treatment that is being contemplated. It can hence be of significant advantage that the results of a test are promptly available. The proposed solutions help with making at least relevant results available in a faster manner.

Thus, according to a further aspect, the method also comprises:
- performing, in-situ using the sensor arrangement connected at a sensor interface, genotyping of the at least one relevant harmful organism from an environmental sample obtained from the crop site, and generating a result signal via the sensor arrangement at the sensor interface;
- processing the result signal, using the processing means, for determining whether the one or more of the at least one relevant harmful organism is present in the environmental sample; thereby detecting via the portable device the presence of any of the at least one relevant harmful organism on the crop site.

More specifically, when viewed from a second perspective, there can also be provided a method for on-site detection of presence of a harmful organism at a crop site, wherein the method is performed using a portable electronic device comprising a processing means and a sensor interface, the method comprising:
- receiving, at the processing means, a crop data; wherein the crop data is indicative of the type of the crop;
- receiving at the processing means a region data; wherein the region data is indicative of the presence of one or more harmful organisms in the region where the crop site is located;
- selecting from a database, using the processing means, a sensor arrangement suitable for selectively genotyping at least one relevant harmful organism; wherein the at least one relevant harmful organism is among the at least one harmful organism, and wherein the selection is performed in response to the crop data and the region data;
- performing, in-situ using the sensor arrangement connected at the sensor interface, genotyping of the at least one relevant harmful organism from an environmental sample obtained from the crop site, and generating a result signal via the sensor arrangement at the sensor interface;
- processing the result signal, using the processing means, for determining whether the at least one relevant harmful organism is present in the environmental sample; thereby detecting via the portable device the presence of any of the at least one relevant harmful organism on the crop site.

As outlined also previously, according to an aspect, the sensor arrangement is selected by the processing means such that the arrangement is also suitable for selectively genotyping at least one benign organism, and the method also comprises:
- processing the result signal, using the processing means, for also determining whether the at least one benign organism is present in the environmental sample.

Thus, it can also be detected via the portable device if any of the at least one benign organism is present on the crop site. The benign organism may for example be a biofungicide.

Accordingly, a method for provide quick detection of relevant harmful organisms on the crop site can be provided. The present teachings thus provide a way to contextualize the types organism according to the crop type before deciding whether they should be detected.

The result signal can be stored either locally, or it may be uploaded to a remote server or database.

As it was discussed previously, the crop data at least partially be acquired automatically, for example via an image recognition system. Also, the region data may at least partially be retrieved from a region database or a remote server. According to another aspect, further information related to the at least one relevant harmful organism may be obtained from the image recognition system. For example, from a captured image, the processing means may, by recognizing distinct features caused by the presence of the organism, at least partially establish that the organism is present on-site. The features may be features of the pest itself, such as shape, size or color of the pest, or they may even be the change in appearance of the crop caused by the harmful organism.

According to an aspect, a combination of the region data, crop data and the result signal is used to prefabricate sensor arrangement for a following season in the future. Thus, based upon detected organisms and local environmental conditions, appropriate sensing devices for the next season can be provided.

According to another aspect, a locus for collecting the environmental sample is determined, via the processing means, dependent upon the regional data, e.g. the season data. According to yet another aspect, a locus for collecting the environmental sample is determined dependent upon the season data and the weather data or prevailing conditions. The processing means can thus improve the sample collection step by selecting sampling location or even a series of sampling locations or sampling path such that the probability of the organism of interest being present in the environmental sample is high. It will be understood that if the organism is indeed present on site, it will be desirable to detect it, so the present teachings can provide a way to achieve a higher reliability of such detection.

Another advantage of the proposed method, besides being easy and fast to use for a non-technical user, can be that analysis and of the result signal can be done locally with reduced processing requirements. Accordingly, it can enable realization of the portable electronic device as a mobile device or a hand-held device or as a low-power battery operated device. Yet another advantage can be that the user, for example, a farmer, can be advised on-site about the detection process, and/or crop treatment, and/or sampling process without a requirement of a fast network or internet connection, for example for uploading sequencing results to cloud, analysis by a remote server and download of recommendation to the electronic device for advising the user. In many cases, this can be a significant advantage, especially for locations with bad internet connection e.g. in rural areas. The portable device can in some cases even perform the outlined functions entirely locally via the database being stored in a local storage medium (such as a computer memory) and by interpreting the result signals via the storage medium. The user may in some cases synchronize the database from such a location where a better network connection is available.

The present teachings can thus provide a method that systematically eliminates irrelevant detection regions, especially in the harmful organism spectrum, thus focusing on the relevant organisms. Detection results can thus be obtained within a time-frame of minutes rather than hours. The proposed teachings are thus particularly suitable for in-situ detection, i.e., measurement or detection done essentially where the environmental sample is collected. In other words, the present invention economizes the effort that is required to identify a relevant harmful organism.

As it was previously mentioned, the sensor arrangement can either be a single device that can selectively detect each of the relevant organisms, or it can be multiple sensors each of which is individually designed for selectively detecting a specific organism different from which the other of the multiple sensors can detect. The multiple sensors can either be simultaneously connectable at the sensor interface, or they may be connectable sequentially, or even in any set of a plurality of sensors that are simultaneously connectable at the sensor interface for detecting a subset of the at least one harmful organism. For example, the sensor arrangement may be a set of sensors that are connected sequentially or one by one at the sensor interface, each sensor detecting a specific harmful organism different from what the other sensors are capable of detecting. As a further example, a plurality or even all of the sensors may be simultaneously connectable at the sensor interface. A specific kind of interface is thus not limiting to the scope of the present teachings.

Accordingly, more generally, there can also be provided a computer-implemented method comprising:
- receiving, at a processing means, a crop data indicative of the type of a crop;
- receiving, at the processing means, a region data indicative of at least one organism that may be present at or around the location of the crop;
- selecting from a database, using the processing means, a sensor arrangement suitable for selectively genotyping the at least one organism; wherein the selection is performed in response to the crop data and the region data.

As discussed previously, the organism may either be a benign organism, or it may be a harmful organism. The advantages of the method are similar to the case above, i.e., when the organism is a harmful organism. These advantages were already discussed in detail. Advantages of the method when the organism is a benign organism is that the processing means is able to select from the database an appropriate sensor for detecting the organism.

Similar to the first perspective, according to an aspect, the method also comprises:
- outputting, via a human-machine interface functionally coupled to the processing means, information about the suitable sensor arrangement.

Further similar to the first perspective, according to a further or an alternative aspect, the method comprises:
- performing, in-situ using the sensor arrangement connected at the sensor interface, genotyping of the one or more organisms from an environmental sample obtained from the crop site, and generating a result signal via the sensor arrangement at the sensor interface;
- processing the result signal, using the processing means, for determining whether the one or more organisms are present in the environmental sample; thereby detecting via the portable device the presence of any of the one or more organisms on the crop site.

Thus, more specifically, similar to the second perspective, there can also be provided a method for on-site detection of presence of an organism at a crop site, wherein the method is performed using a portable electronic device comprising a processing means and a sensor interface, the method comprising:
- receiving, at the processing means, a crop data; wherein the crop data is indicative of the type of the crop;
- receiving at the processing means a region data; wherein the region data is indicative of the presence of one or more organisms in the region where the crop site is located;
- selecting from a database, using the processing means, a sensor arrangement suitable for selectively genotyping the at least one organism; wherein the selection is performed in response to the crop data and the region data;
- performing, in-situ using the sensor arrangement connected at the sensor interface, genotyping of the one or more organisms from an environmental sample obtained from the crop site, and generating a result signal via the sensor arrangement at the sensor interface;
- processing the result signal, using the processing means, for determining whether the one or more organisms are present in the environmental sample; thereby detecting via the portable device the presence of any of the one or more organisms on the crop site.

As discussed previously, the organism may either be a benign organism, or it may be a harmful organism.

According to an aspect of any of the previous perspectives, the method further comprises:
- generating, via the processing means, a processed result signal indicating the presence of the one or more organisms in the environmental sample.

According to an aspect, the processed result signal is generated by analyzing data, for example raw measurement data, from the result signal generated by the sensor arrangement. Alternatively, the processed result signal could even be essentially a copy of the result signal.

The term environmental sample encompasses any kind of sample that can be used to detect the presence of the organism. Accordingly, it is not essential that the environmental sample comprises a portion of the crop. The environmental sample, thus, may or may not comprise a portion of the crop. The environmental sample may be collected from any one or a combination thereof of: air, water, inorganic or organic matter from the crop site. For example, the environmental sample may be a soil sample obtained from the crop site, and/or it may be a foliage or other organic matter such as mulch, humus or composted matter obtained from the crop site. The environmental sample may even be pollen collected either directly from the crop or on-site from air. It will thus be appreciated that the environmental sample can be any one or more of the: whole plant, plant parts such as leaf, root, flower, pollen, soil samples, spore collections (e.g., on filter paper). A foliage or leaf sample may comprise a part of the crop, or it may just be a weed foliage or a weed plant part. When a sample is collected from air on-site, indicators of the organism such as spores, traces, etc. may be collected. In some cases, the sample may comprise the organism in the form of an insect or a part thereof. According to an aspect, a soil sample or a sample of remaining biomass on-site, e.g., of a crop or weed from the previous season, is used to identify at least one organism surviving or hibernating in the soil or biomass. Accordingly, the processing means may determine at least one harmful organism which can infect the next season crop. Especially for detecting microscopic organisms, any of the aforementioned sample types may be collected either alone or in combination for detection of such microscopic organisms in the sample.

The selected sensor arrangement is connected at the sensor interface which functionally connects at a given time at least one of the sensors of the sensor arrangement to the processing means. The sensor interface can either be a hardware interface. This, for example, can be a case when at least one of the sensors in the sensor arrangement is in the form of a cartridge that is plugged into an electronic device comprising the processing means. The sensor arrangement may have terminals or pins that form a part of the sensor interface by functionally connecting at a given time at least a part of the sensor arrangement to the processing means. This can be advantageous especially in cases when the electronic device is a handheld device by doing so the sensor arrangement becomes attached to the handheld device and thus more convenient for on-site measurements.

In some cases, the sensor interface may even be a wireless interface. Accordingly, the at least one of the sensors may functionally connect wirelessly to the processing means. This can be advantageous especially in cases when the electronic device is a larger unit, perhaps not suitable of being a handheld device. In which case, the wireless sensor can be advantageous as a lightweight unit for collecting environmental samples on-site and performing in-site detection of harmful organisms from those samples. The specific examples of wired and wireless sensors are provided in a non-limiting sense. A combination of a wired and wireless types is also possible. It will be understood that specific realization of such a sensor is not essential for the scope or generality of the present teachings.

Subsequent to the environmental sample being introduced for detection to the selected sensor arrangement, the sensor performs in-situ detection and optionally quantification when using a suitable sensor arrangement, of the one or more harmful organisms in the sample. The result of the detection is generated in the form of a result signal. The result signal can be a signal comprising outputs from different sensors in the sensor arrangement, which will be the case when detecting multiple different organisms. The sensor outputs can either be generated at the same time, or at different time from generating other sensor outputs, which will be the case when the sensor arrangement comprises sensors which are connected sequentially to the sensor interface.

The processing means processes the result signal to determine whether the one or more relevant harmful organisms are present (i.e., have been detected by the respective sensors of the sensor arrangement) in the environmental sample. As it will be understood already, the one or more organisms are the organisms of interest that were narrowed down by combining the crop data with the region data, especially when the one or more organisms are harmful organism. Accordingly, as discussed, the sensor arrangement was selected based on both those data. The method as proposed can thus shorten the detection time. Furthermore, costs can also be saved by use of a targeted sensor that detects specific variety of harmful organisms as found to be of interest by combining the crop data with the region data, as compared to using, for example, sequencing measurements on the entire field. Hence, the proposed teachings are also suitable for realizing a map of organisms. The map may reflect geographical distribution of the one or more relevant organisms, i.e., one or more of the at least one relevant harmful organism and/or at least one benign organism. Accordingly, through the results collected from a plurality of detections performed over a given area, a map representative of the spread of any of the one or more relevant organisms over the area may be obtained. The map may even be an internal map of a greenhouse or even a collective map for a plurality of greenhouses. Greenhouses are enclosed or semi-enclosed structures used for cultivating crops such as vegetables and fruits. Such a greenhouse map can be used to track which greenhouse is infected by a harmful organism e.g. resistant mutant or virus such that quarantine measure and/or treatment may be determined or planned.

Those skilled in the art will realize that a faster detection as realized by the proposed method can also enable generation of a higher resolution map. Furthermore, the lower cost of the sensor can also be an enabling factor for a generation of such a detailed map. Thus, according to another aspect of any of the perspectives, the method also comprises:
- generating a site result data, using the processing means, by combining the processed result signal and the location data of the crop site where the environmental sample was collected;
- storing, in at least one database, the site result data;
- combining data from a plurality of the site result data to obtain a field map representative of the territorial spread of any of the one or more relevant organisms.

The location data may either be a part of the region data, or it may be received from an alternative source, for example, a geolocation system. Geolocation systems such as GPS and GALILIO are available as modules that can be integrated for example in the handheld device, and/or in the lab-on-chip device. The processing means may thus obtain the location data of the crop site from such a module. Those skilled in the art will appreciate that alternative geolocation methods and devices such as radio navigation techniques can also be applied. Any technique which provides adequate spatial resolution of location for being able to distinguish between two territorially separated environmental samples can be applied with the proposed teachings.

The field map thus obtained can provide finer resolution of organism spread data. This indeed can have several advantages, for example, any variation in the organism population spread over the field area may be recognized. Accordingly, zones of high and/or low organism activity may be obtained. This can for example provide information on how effective a treatment has been, or even provide information of the direction from which the harmful organism population infestation started. In some cases, organism hotspots and/or cold spots can even be obtained. By a hotspot, it is here meant an area where any one of the one or more relevant organisms are more active as compared to the neighboring area. In some cases, these hotspots may be related to the conditions that are locally prevailing at the respective sites where the organisms are more active. A possibility to recognize the hotspots for relevant harmful organisms can enable an appropriate treatment of such hotspots. Similarly, colds spots can also be recognized. By a cold spot, it is here meant an area where at least one of the one or more relevant organisms are not active, or less active as compared to the neighboring area. Similarly, in some cases, these cold spots may be related to the conditions that are locally prevailing at the respective sites where the organisms are less active. It may be possible that the organism activity may be influenced by replicating such conditions. For example, in some cases it may be possible to reduce or even eliminate harmful organisms in the rest of the crop by replicating the conditions prevailing locally in a cold spot of harmful organisms. The recognition of either or both of one or more hot spots or cold spots of relevant harmful organisms may offer advantages in terms of reduced or more effective use of active ingredients such as pesticides, fungicides, insecticides or herbicides. Similarly, recognition of one or more hot spots or cold spots of benign organisms may be beneficial for making the overall biocontrol more effective.

The prevailing conditions that have influence in a hotspot or a cold spot may include any one or more of the determining factors: temperature, moisture level, pH value, sunlight and/or wind conditions.

Another important advantage of such a map is that a better overview of the density of the organisms can be obtained, which can provide valuable information of the magnitude of infestation. This can be important for planning a treatment of such areas according to the organism activity. According to another aspect, the method further comprises:
- determining, by analyzing the field map via the processing means, at least one site where another measurement or detection is required.

The another measurement may either be the same kind of detection that was performed earlier, or it may be another measurement. For example, the another measurement may be a measurement of at least one parameter to determine the prevailing condition. The another measurement may even be a sequencing measurement which is recommended to obtain further information regarding the prevailing condition.

It will be appreciated that by implementing any of the above aspects of acquiring crop data and/or region data, the portable electronic device may be at least partially automated. In some cases, the portable device may be fully automated, for example, in the form of a robot that performs a plurality of in-situ measurements at the crop location. For example, the computer-implemented method including the performance of genotyping the organism, creating a result signal etc., may be carried out online on a agricultural machine, e.g. a tractor. The result signal, the processed result signal, or the map generated thereof may then be applied simultaneously to adapt any agricultural measures taken by the agricultural machine. For example, the machine may use the information conveyed by these signals or the map to select a pesticide and / or the dose rate of a pesticide over the whole field, or dependent on the section of the field according to said cold and hot spots, i.e. in response to the information conveyed by the result signal, the processed result signal, or the map.

The robot (e.g. the agricultural machine) may for example, automatically fetch region data, combine the region data with the crop data - the latter of which can even be automatically determined. The result signals obtained from the plurality of measurements can be stored either locally, or they may be uploaded to a remote location using a communications network, for example, via a wireless network connection. The result signals may also include data related to at least one benign organism.

According to another aspect, the method also comprises:
- determining, in response to any of the crop data, region data, and the selected sensor arrangement, a sampling method for collecting the environmental sample.

The determination of the sampling method can be done by the processing means either using the same database as used for selecting the sensor arrangement, or it may be another database. According to an aspect information related to the sampling method is outputted via the HMI. This can have an advantage that the on-site analysis can be done by a non-expert user. For example, the farmer when performing the measurements on their field does not require to know in advance how to perform the sampling. The portable device can thus guide the user through the correct way of collecting the sample. There can be different kinds of sampling methods, which can vary significantly based on the kind of crop, the organism, the sensor arrangement, or even their combination. Sampling may involve any of the: collecting a specific portion of the foliage or another part of the crop, extracting the sample via a hole punched in the foliage, collecting all or a portion of the organism, etc. Thus, if the sampling is not properly performed, the detection of the organism may not be reliable. In some cases, sampling or sample preparation that is required can be so specific that expert knowledge can be required, for example, NaOH based extraction and Whatman FDA cards. Thus, the proposed teachings, also when combined with the rest of the aspects, makes the process easy for the user by guiding the user through the sampling method and process required. The user can even be guided through the process, for example through sample collection process and/or sample preparation process. It can thus be avoided that the sampling is done by an expert user or a requirement of advance knowledge of any of such processes.

According to yet another aspect, the sampling and/or sample preparation is done automatically by the portable device, for example, in the form of a robot. The robot may automatically perform sampling according to the sampling method selected by the processing means.

According to an aspect, the method also comprises:
- determining, in response to the result signal or the processed result signal, a treatment method for controlling at least one of the at least one relevant harmful organism present on the crop site; and optionally providing the information on the treatment method as a recommendation to the user.

It will be understood that the treatment method is thus determined by the processing means by analyzing the result signal or the processed result signal. The determination of the treatment method can be done by the processing means either using the same database as used for selecting the sensor arrangement, or it may be by using another database. The database may contain additional information on the efficacy of different agricultural products on a given harmful organism. For example, the database may contain information on suitable pesticides to combat a harmful organism that is resistant to a particular or several pesticides. Such information may even be regularly updated, e.g. as resistances change in type, significance, and local spreading.

The recommended treatment to the user may also entail product recommendations. These product recommendations may be chosen by economic efficacy, biological efficacy, availability and the like. The recommended product may even be ordered automatically from a vendor. For example, the method may comprise a step in which the availability of the stocked recommended product at the farm or the organizational unit to which the field belongs to where the crop plant is growing or is intended to be grown. Said information may be stored at a database. Thus, the method may comprise receiving information from a database on the local availability of the recommended treatment, i.e. the availability of the recommended product. If the data indicates that the product stocks are low (e.g. below 10% of capacity) or empty, the method may comprise the further step of automatically ordering the product from a vendor. Of course, the ordering may alternatively be effected by the user having received the information on the recommended treatment.

According to an aspect the recommendation information related to the treatment method is output via the HMI. This can have an advantage that the on-site analysis can be done by a non-expert user and the recommendation for treating the any of the relevant harmful organisms may be provided to the user on the spot. The treatment method may even be determined in response to a plurality of result signal or processed result signal. The plurality of such signals may be either previous and recent results from the same site or a nearby site, or the plurality of signals may be a combination of signals related to adjacent sites, or a combination of previous signals and signals from adjacent sites.

According to another aspect when a field map is generated, the method further comprises:
- determining, by analyzing the field map via the processing means, a treatment method for controlling at least one of one or more harmful organisms present on the crop site.

It will be appreciated that by doing so, the processing means can recognize which areas of the crop can require which kind of treatment. A prospective treatment can thus be optimized according to spread of the organism over the crop area or parts thereof. Another combined advantage is that the treatment can be selected according to the density of the organism spread. Problematic areas of the crop can thus be treated appropriately.

According to another aspect, the method also comprises:
- performing a treatment in accordance to the treatment method to control the at least one of the at least one relevant harmful organism detected on the crop site.

The treatment can either be performed using the portable electronic device, or it may be performed using a separate treatment device functionally coupled to the portable electronic device.

It will be understood that the treatment may be a spray program involving spraying of one or more active ingredients to control the at least one of one or more harmful organisms. For example, the treatment may involve spraying of a fungicide on at least a portion the crop or crop site to control a fungus that has been detected by the processing means. The active ingredient may be a chemical compound and/or it may be a biological compound used for example, for biocontrol. In some cases, the treatment can even involve a physical removal of the organism from the crop or the crop site.

According to an aspect, at least a portion of the result signal is also fed back to the database, to improve the database further. The portion of the result signal may include the detected organism and genotype of the organism. The portion may even include the treatment recommendation that was determined and/or applied to control the organism. From subsequent measurements done at or around the same site or along the same or similar locus, the processing means may determine whether the treatment has been effective in controlling the harmful organism. This can even help identify new resistant mutants which were not detected previously. Similarly, there can also be provided an electronic device comprising the processing means configured to perform the method steps disclosed herein.

More specifically, similar to the first perspective, there can be provided an electronic device comprising a processing means, wherein the processing means is configured to receive:
- a crop data indicative of the type of a crop; and
- a region data indicative of at least one harmful organism that may be present at or around the location of the crop, wherein
the processing means is configured to select a sensor arrangement from a database, wherein the sensor arrangement is suitable for selectively genotyping at least one relevant harmful organism, the at least one relevant harmful organism being among the at least one harmful organism, and wherein the selection is performed in response to the crop data and the region data.

According to an aspect, the electronic device further comprises a human-machine-interface ("HMI") functionally coupled to the processing means. The HMI is configured to output information related to the suitable sensor arrangement. The information is output for the user.

According to another aspect, the sensor arrangement is configured to perform in-situ genotyping of the at least one or more relevant harmful organisms from an environmental sample obtained from the crop site; the sensor arrangement being functionally connected at a sensor interface, and wherein the electronic device is further configured to generate a result signal via the sensor arrangement at the sensor interface.

According to further an aspect, the processing means is configured to process the result signal for determining whether the at least one or more relevant harmful organisms is present in the environmental sample; thereby detecting via the electronic device the presence of any of the at least one or more relevant harmful organisms on the crop site.

The electronic device is preferably a portable device or a portable electronic device.

More specifically, there can be provided a portable electronic device comprising a processing means and a sensor interface, wherein the processing means is configured to:
- receive a crop data indicative of the type of a crop; and
- receive a region data indicative of at least one harmful organism that may be present at or around the location of the crop;
- select from a database a sensor arrangement suitable for selectively genotyping at least one relevant harmful organism, the at least one relevant harmful organism being among the at least one harmful organism, wherein the selection is performed in response to the crop data and the region data; and wherein
the sensor arrangement is configured to perform in-situ genotyping of the at least one relevant harmful organism from an environmental sample obtained from the crop site; the sensor arrangement being functionally connected at a sensor interface, and wherein the electronic device is further configured to generate a result signal via the sensor arrangement at the sensor interface, and wherein the processing means is configured to process the result signal for determining whether the at least one relevant harmful organism is present in the environmental sample; thereby detecting via the electronic device the presence of any of the at least one relevant harmful organism on the crop site.

According to another aspect, the electronic device is further configured to process the result signal via the processing means for further determining whether an at least one benign organism is present in the environmental sample.

According to another aspect, the electronic device also comprises a geolocation module for determining the geographical location of the crop site. According to another aspect, the electronic device also comprises a communication module functionally connected to the processing means. The communication module may be used for connecting to a communications network and/or database. For example, the communication module may be a wireless network connection module, and/or a Bluetooth^{®} module or such.

According to another aspect, the electronic device is configured to:
- generate a site result data, using the processing means, by combining the processed result signal and the location data of the crop site where the environmental sample was collected;
- store, in at least one database, the site result data;
- combine data from a plurality of the site result data to obtain a field map representative of the territorial spread of any of the at least one relevant harmful organism.

According to another aspect, the electronic device is further configured to:
- determine, by analyzing the field map via the processing means, at least one site where another measurement or detection is required.

According to another aspect, the electronic device is further configured to:
- determine, in response to any of the crop data, region data, and the selected sensor arrangement, a sampling method for collecting the environmental sample.

According to an aspect, the electronic device is further configured to:
- determine, in response to the result signal or the processed result signal, a treatment method for controlling at least one of the at least one relevant harmful organism detected on the crop site; and optionally
- providing the information on the treatment method as a recommendation to the user.

According to an aspect, the electronic device is further configured to:
a) receive, from a database, information on pesticides effective to combat harmful organisms that are relevant to at least one pesticide;
b) generate a recommendation on a recommended treatment method to the user, e.g. a product recommendation, which is based on the information a) stored in the database and the result signal or the processed result signal.

According to an aspect, the electronic device is further configured to:
c) receive, from a database, information on the local availability of the recommended treatment method;
d) in case of no or low availability, effect the shipment of the recommended product from a vendor.

According to another aspect, the electronic device is further configured to:
- determine, by analyzing the field map via the processing means, a treatment method for controlling at least one of the at least one relevant harmful organism detected on the crop site.

According to another aspect, the electronic device is further configured to:
- perform a treatment in accordance to the determined treatment method.

The treatment is performed at least partially on the crop site.

The processing means may be a general-purpose processing device such as a microprocessor, microcontroller, central processing unit, or the like. More particularly, the processing means may be a CISC (Complex Instruction Set Computing) microprocessor, RISC (Reduced Instruction Set Computing) microprocessor, VLIW (Very Long Instruction Word) microprocessor, or a processor implementing other instruction sets or processors implementing a combination of instruction sets. The processing means may also be one or more special-purpose processing devices such as an ASIC (Application-Specific Integrated Circuit), an FPGA (Field Programmable Gate Array), a CPLD (Complex Programmable Logic Device), a DSP (Digital Signal Processor), a network processor, or the like. The methods, systems and devices described herein may be implemented as software in a DSP, in a micro-controller, or in any other side-processor or as hardware circuit within an ASIC, CPLD, or FPGA. It is to be understood that the term "processing means" or processor may also refer to one or more processing devices, such as a distributed system of processing devices located across multiple computer systems (e.g., cloud computing), and is not limited to a single device unless otherwise specified.

When viewed from another perspective, there can also be provided a computer program comprising instructions which, when the program is executed by a processing means of an electronic device, cause the electronic device to carry out the method steps herein disclosed.

For example, there can be provided a computer program comprising instructions which, when the program is executed by a processing means of an electronic device, cause the electronic device to:
- receive, at the processing means, a crop data indicative of the type of a crop;
- receive, at the processing means, a region data indicative of at least one harmful organism that may be present at or around the location of the crop;
- select from a database, via the processing means, a sensor arrangement suitable for selectively genotyping the at least one relevant harmful organism; wherein the at least one relevant harmful organism is among the at least one harmful organism, and wherein the selection is performed in response to the crop data and the region data.

As a further example, there can also be provided a computer program comprising instructions which, when the program is executed by a processing means of an electronic device comprising a sensor interface, cause the electronic device to:
- receive, at the processing means, a crop data; wherein the crop data is indicative of the type of the crop;
- receive at the processing means a region data; wherein the region data is indicative of the presence of one or more harmful organisms in the region where the crop site is located;
- select from a database, using the processing means, a sensor arrangement suitable for selectively genotyping the at least one relevant harmful organism; wherein the at least one relevant harmful organism is among the one or more harmful organisms, and wherein the selection is performed in response to the crop data and the region data;
- perform, in-situ using the sensor arrangement connected at the sensor interface, genotyping of the at least relevant harmful organism by analyzing an environmental sample obtained from the crop site, and generating a result signal via the sensor arrangement at the sensor interface;
- process the result signal, using the processing means, for determining whether the at least one relevant harmful organism is present in the environmental sample; thereby detecting via the portable device the presence of any of the at least one relevant harmful organism on the crop site.

When viewed from yet another perspective, there can also be provided a computer-readable data carrier having stored thereon the computer program herein disclosed. Accordingly, there can also be provided a non- transitory computer readable medium storing a program causing a suitable electronic device to execute any method steps herein disclosed.

A computer-readable data carrier includes any suitable data storage device on which is stored one or more sets of instructions (e.g., software) embodying any one or more of the methodologies or functions described herein. The instructions may also reside, completely or at least partially, within the main memory and/or within the processor during execution thereof by the computer system, main memory, and processing device, which may constitute computer-readable storage media. The instructions may further be transmitted or received over a network via a network interface device.

The computer program for implementing one or more of the embodiments described herein may be stored and/or distributed on a suitable medium, such as an optical storage medium or a solid state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network.

When viewed from another perspective, a data carrier or a data storage medium for making a computer program element available for downloading can be also provided, which computer program element is arranged to perform a method according to one of the previously described embodiments.

The word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

Example embodiments are described hereinafter with reference to the accompanying drawings.
- FIG. 1: shows a block diagram representation of a portable electronic device deployed in a crop field.
- FIG. 2: shows a spectrum sketch for illustrating the targeted approach of detection
- FIG. 3: shows a flowchart of a sensor arrangement selection method
- FIG. 4: shows a flowchart of a selective genotyping performed after selection

### Detailed description

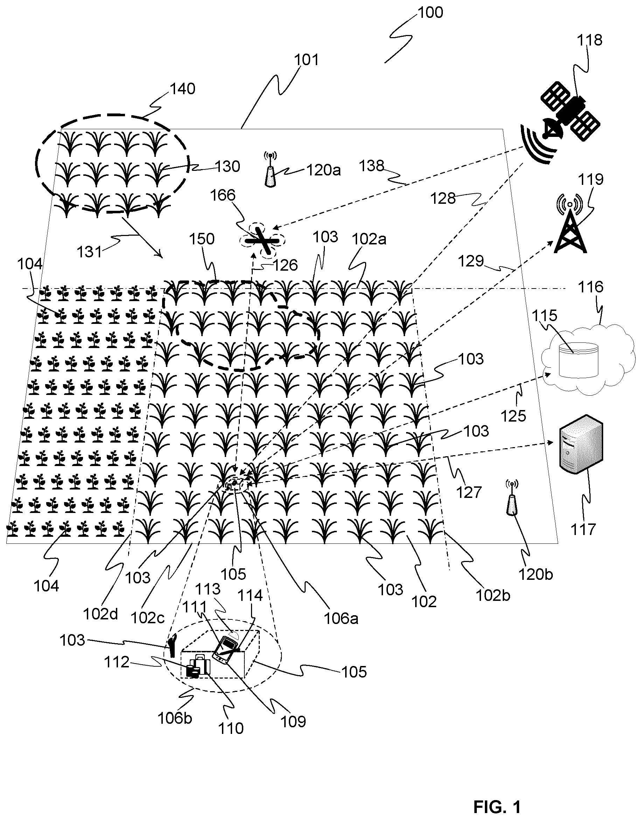

FIG. 1 shows a block diagram 100 illustrating a scenario in which some of the present teachings are applied. In a non-limiting sense, a terrestrial geographical section 101 is shown which may represent a geographical area on land. On the section 101, a crop comprising of plants 103 is planted. The crop, or plants 103, is planted on a field 102, or a geographical area, defined by a boundary 102a-d. On the left side 102d of the field 102 is shown planted another crop which comprises another plants 104 which are different from the plants 103 on the field 102. A detection system 105 pursuant to an aspect of the present teachings is shown located at a site 106a on the field 102. A zoomed-in view 106b is also shown of the site 106a showing an example of the detection system 105 in more detail. The detection system 105 comprises an electronic device 109, which is shown as a handheld device. The detection system 105 also comprises a plurality of sensors or sensor arrangement 112, which for example, may be stored in a kit 110. Any of the sensors 112, for example, a selected sensor or a sensor arrangement 113 can be connected to the electronic device 109 via a sensor interface. The sensor interface can be wireless or wired. The electronic device 109 comprises a human-machine-interface ("HMI"), which here is shown to include a stylus 114 and a display 111 for a user to interact with the device 109. The HMI may even include an audio device such as a loudspeaker. The electronic device 109 also comprises a processing means or computer processor.

The detection system 105, or more specifically, the electronic device 109 may comprise a geolocation means or a geolocation module for receiving location data. The location data is representative of the geographical location of the site 106a. For example, the device 109 may receive the location data through one or more satellites 118. Geolocation systems such as GPS and GALILIO are available as modules that can be integrated for example in the device 109, and/or even in the sensors 112, 113. Alternatively, or in addition, the location data may be obtained from one or more cellular network 109. Alternatively, or in addition, the location data may be obtained from one or more wireless stations or beacons 120a-b. The system 105 or preferably the electronic device 109 may connect to at least one network, for example, via a data connection 129 with the cellular network 119, and/or via a wireless connection with any of the wireless stations 120a and/or 120b.

The system 105 or the device 109 also has a functional connection 125 to at least one database 115. In some cases, the database 115 can be at least partially a local database, i.e., comprised within a memory of the system 115 or more specifically that of the device 109. In such cases, the database connection is at least partially internal to the system 105 or the device 106. In some cases, the database 115 can be at least partially a remote database, i.e., located at another location as compared to the location 106a of the system 105. In such cases, the database connection 125 is at least partially external to the system 105. Hence the database connection 125 may be established via the cellular network 119 and/or via one or more wireless station 102a, b. The at least one database 115 may even at least partially be on a cloud service 116. In some cases, the external database connection 125 can be established away from the test location 106a. This is relevant when for example, a cellular network or a wireless connection cannot be made from the test site. In such cases, the database 115 can be an internal database that can be synchronized with a remote database when a network connection is available. The synchronization can either be made directly via a cellular connection 129 and/or a wireless network 120 when it is available, or the synchronization can be done indirectly by a connection 127 to a server 117. In some cases, the server connection 127 is a wired connection between the device 109 and the server 117, while in other cases it can even be a wireless connection, for example through the data connection 129 or wireless 120. In some cases, the remote database 115 may at least partially reside on the server 117, or even the server 117 may provide access to the remote database 115 that may be on another location.

When the system 105 is deployed at a crop site, for example the site 106a, the processing means receives crop data which is indicative of the type of crop, i.e., the type of plants 103. The crop data may either be entered by the user, for example using the HMI, and/or it may be acquired automatically via a crop sensor, and/or even via previous measurements. The crop sensor may, for example, be a part of an image recognition system. The processing means also receives region data which is indicative of at least one harmful organism that may be present at or around the location of the crop 103. For this purpose, the region data may provide information on which organisms are or have been present within a certain distance of the field 102. In some cases, the region data may be at least partially obtained from the server 117, and/or the database 115, and or the cellular network 119, and/or the wireless network 120. The region data may even comprise the location data. In some cases, the region data is available from one or more public databases or an internet service. The region data may even comprise weather data, for example intensity and direction of wind 131.

If an infected area 140 lies between the path of the wind 131 upstream of the field 102, then there can be a higher probability that harmful organisms from infected plants 130 have been transported to at least a portion of the crop 103. The certain distance of the field 102, which is to be evaluated for relevance of detection of the harmful organism may thus be adapted according to the prevailing conditions, such as the wind 131 or the history thereof.

In some cases, the other crop 104 may even have been infected by another harmful organism. In such cases, the region data may also comprise information related to the another harmful organism. It is however possible that the another harmful organism does not infect the crop plants 103. It can therefore be useless to treat the crop 103 for the another harmful organism that only affects the other crop 104, or more specifically, that does not affect the crop 103. Such an organism can be considered an irrelevant harmful organism for the crop 103.

Similarly, there may be further organisms that are harmful to the crop 103, however such organisms have not been detected within the certain distance of the field 102 or even the site 106a. It thus may not be sensible to detect such further organisms either. Such organisms can also be considered irrelevant harmful organisms for the crop 103.

Pursuant to the present teachings, by combining the crop data with the region data, the processing means is thus able to automatically determine the harmful organisms of interest, or at least one relevant harmful organism if both the crop data and the region data indicate the same harmful organism. It will be appreciated that if the at least one harmful organism indicated by the region data does not correspond to those organisms that can affect the crop, none of the at least one harmful organism will be the relevant harmful organism, hence no detection will be required to be performed. Similarly, if all of the at least one harmful organism indicated by the region data correspond to the respective organisms that can affect the crop, all of the at least one harmful organisms will be the relevant harmful organisms, and hence be recommended for detection. So, when at least one relevant harmful organism is indicated by combining the crop data and the region data, the processing means can thus automatically select a sensor arrangement comprising one or more sensors, each of which sensors are targeted to detect a specific individual organism of interest different from the organisms that other sensors in the sensor arrangement can detect. It can thus be made possible to determine a sensor arrangement that is optimized to detect the organisms of interest. or relevant organisms. The user is neither required to be an expert in organism, crops, or their detection.

When the selected sensor arrangement 113 is used to perform the detection, the device 109 can guide the user via HMI through the sampling process. The sampling process may differ dependent upon the crop and/or the relevant organism to be detected. The sampling process may even depend upon the selected sensor arrangement 113. The user is hence not required to be an expert or a biologist. It will also be appreciated that the method explained above is also suitable to be implemented as an autonomous process, for example, the system 105 may be a robotic system that after automatically selecting the sensor arrangement 113, interfaces the arrangement 113 to the processing unit, acquires one or more environmental samples from the site 106a, selectively genotypes the environmental sample, generates a result signal via the sensor arrangement, processes the result signal using the processing means to determine whether the one or more harmful organisms are present in the environmental sample obtained from the crop site 106a. The robotic system may then pick another site for detection at the another site. The present teachings are thus suitable both for detection performed by a portable device operated by a user, or by an at least partially autonomous process.

In response to a positive detection of the harmful organism which has been detected at a site, the processing means may determine a suitable treatment for controlling the harmful organism. If more than one relevant harmful organisms are detected at the site, the processing means may determine a treatment that is optimized for treating more than one organism harmful organisms on the site. This can help minimize the amount and number or active ingredients for controlling the organisms rather than treating each harmful organism separately. Such an optimization of treatment can also be a significant advantage for an inexperienced user. This can thus have cost and environmental advantages. The treatment can for example be a spray program than involved spraying the infected plants with a specific active ingredient or mixture thereof. The processing means can thus select a treatment that is suited according to the crop and organism. Furthermore, the processing means can even select the appropriate treatment that is optimized according to the weather and/or season. The system 105 or the device 109 can even guide the user through the treatment process, which may include any of the: name of the suitable treatment product, time of start of the treatment using the product, dose rate, volume of application, number of applications of the product or even a combination of treatment products dependent upon the one or more result signals from the site or the neighboring or adjacent sites. Rather than risking too much or too scarce application of the product, the user can be guided through an optimized application according to the result data from one or more result signals. Again, it will be appreciated that rather than guiding the user, an autonomous treatment system can also be used that is operated similar to as outlined above, in response to the result data, and even location data. The treatment system may either be the same system 105, or it may be a separate system such as at least one drone 166. The drone 166 may also comprise a location detection means such as a geolocation module that acquires a location signal 138 from one or more satellites 118. The drone may even obtain location data via the detection system 105, or via cellular network 119 or wireless network 120, as was explained earlier in context of the system 105.

In FIG. 1 it is also shown an infected portion 150 of the crop plants 103. The portion 150 may have become infected by pathogens which were borne by the wind 131 from the infected area 140. Such portion 150 may, for example, be determined/detected by combining the result signals from a plurality of crop sites. By further combining the result signal with the location data of the respective sites, a map representing geographical distribution of the one or more relevant organisms may be obtained. The infected portion 150 may even be called a hot-zone of the organism that is present in the portion 150. Such a map may be useful in targeted treatment of the infected plants within, and in some cases also around, the hot-zone 150. Subsequent detections can be made on the same or similar sites on the field 102 for tracking the effectiveness of the treatment and/or spread. Further treatments can thus be optimized. This can also have cost and ecological benefits by avoiding treatment in response to the harmful organism being detected controlled. These benefits are synergistically linked by making it possible to perform a targeted detection in a quick way such that frequent and higher density measurements can be afforded.

In some cases, the system 105 automatically places an order of one or more sensors in the selected sensor arrangement should the one or more sensors not be available in the system 105, or are expected to be exhausted for detections that are planned in the future. In some cases, the system 105 can automatically place order of the treatment product as required to control the detected one or more relevant harmful organism.

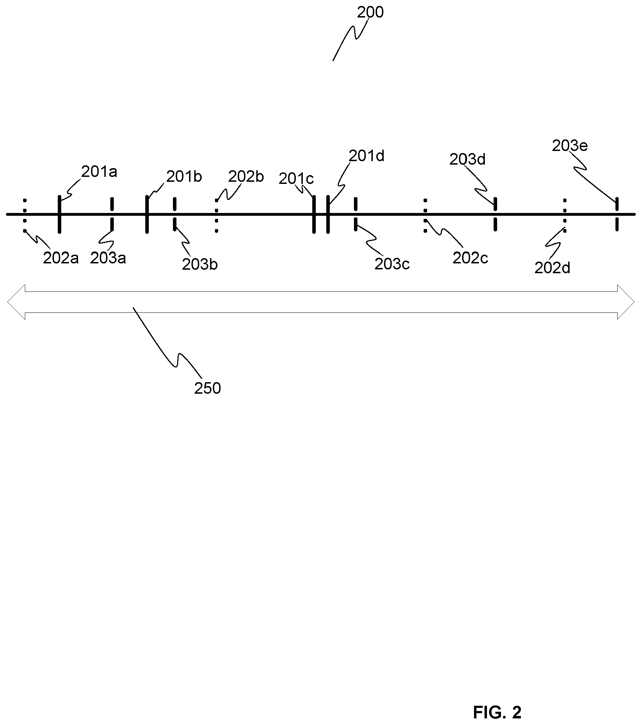

FIG. 2 shows a symbolic representation 200 of the targeted approach of selection and for detection of organisms as proposed in the present teachings. Along the entire spectrum of organism genome 250, different organisms can lie in different places in the spectrum. The organism spectrum can include organisms that can affect the crop 103, for example, the first group 201 of organisms and a second group 202 of organisms. In addition, there can be a third group 203 of organisms that cannot affect the crop 103. If the region data indicates that organisms 201 and 203 may be present at or around the location of the crop 103 or the field 102, the processing means can combine the crop data with the region data and as a result automatically exclude the organisms 203 from the scope of detection. As a result, the processing means will pick only the first group of organisms 201 for detection. and thus define them as relevant harmful organisms. Accordingly, the second group of organisms 202 also be excluded, as irrelevant organisms, from the scope of detection as these organisms have are not present at or around the location of the crop. Accordingly, the processing means, in response to the crop data and the region data, selects from the database 115 the sensor arrangement 113 suitable for selectively genotyping the relevant harmful organisms 201a - d. It will be appreciated that the term group may even mean a single organism and does not need to be a grouping of organisms. Accordingly, each specific organism may also be checked for relevance by the processing means for defining the scope of detection. The term scope of detection means the organisms that have been selected for detection by the processing means by combining the crop data with the region data. The sensor arrangement 113 is thus selected such that it is able to selectively genotype these selected organisms.

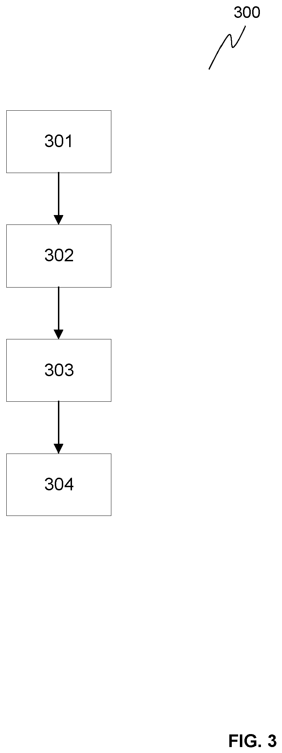

FIG. 3 shows a flowchart 300 for selecting a sensor arrangement. In an initial step 301, crop data is received at the processing means. The crop data indicative of the type of the crop. For example, crop data indicates that the crop plants 103 are *wheat.* The crop data may even further specify the type of crop, for example, that the crop plants 103 are of the type *Durum wheat.* Then is a subsequent step 302, region data is received at the processing unit. The region data is indicative of at least one harmful organism that may be present at or around the location 106a of the crop. In another subsequent step 303, the processing means selects a sensor arrangement 113 from a database 115 in response to the crop data and the region data. The sensor arrangement is suitable for selectively genotyping the at least one relevant harmful organism. It will be appreciated that the sequence of the initial step 301 and the subsequent step 302 may be interchanged. The processing means may still be able to select the sensor arrangement 113 from the database 115 by combining the crop data and the region data. In an optional subsequent step 304, information related to the selected sensor arrangement 113 is outputted via an HMI such as a graphic display.

The flowchart 300 can be implemented on a suitable electronic device 109 comprising the processing means and a sensor interface for functionally connecting the sensor arrangement 113 to the processing means. Accordingly, the detection system 105 may be realized.

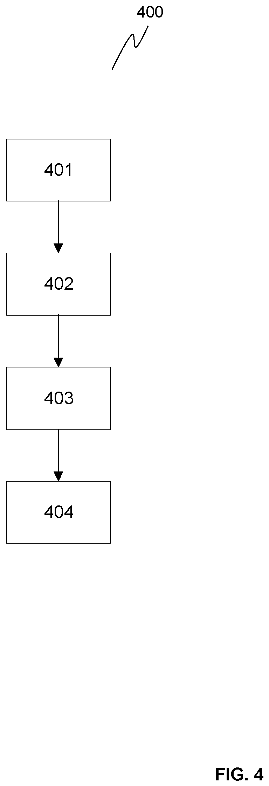

FIG. 4 shows another flowchart 400, which may follow, for example the steps 301 - 303 of, the first flowchart 300. Step 304 may optionally be present as well. In a first next step 401, selective genotyping of an environmental sample is performed using the sensor arrangement 113 functionally connected at the sensor interface. The environmental sample obtained from the crop site 106a. A result signal is generated at the sensor interface by performing the selective genotyping via the sensor arrangement 113. In a further next step 402, the result signal is processed by the processing unit for determining whether the one or more relevant harmful organisms are present in the environmental sample. The electronic device 109 can thus detect if any of the one or more relevant harmful organisms are present or not on the crop site 106a. As an optional further next step 403, the processing means also determines from the result signal whether the at least one benign organism is present in the environmental sample. The determination can be in the form of a processed result signal generated by the processing means, the processed result signal indicating the presence of the one or more organisms in the environmental sample. As a further optional step, either with or without the step 403, the processing means generates a site result data by combining the processed result signal and the location data of the crop site 106a where the environmental sample was collected. Also optionally, the processed result signals collected from detections performed on a plurality of sites can be used to generate a map representative of the spread of any of the one or more relevant organisms over the geographical area covered by the plurality of sites. In further optional steps, determination can be made for suitable sampling steps, treatment and further measurements. Optionally, the user can be guided through each or all of the steps, or further optionally, they may be implemented in an autonomous manner.

Various examples have been disclosed above for a method suitable for on-site detection, an electronic device for on-site detection, and a computer software product implementing any of the relevant method steps herein disclosed. It will further be appreciated that aspects from the method and product embodiments discussed herein may be freely combined.

## Claims

1. A computer-implemented method comprising:
- receiving, at a processing means, a crop data indicative of the type of a crop;
- receiving, at the processing means, a region data indicative of at least one harmful organism that may be present at or around the location of the crop;
- selecting from a database, using the processing means, a sensor arrangement suitable for selectively genotyping at least one relevant harmful organism; wherein the at least one relevant harmful organism is among the at least one harmful organism, and wherein the selection is performed in response to the crop data and the region data,
wherein the sensor arrangement is suitable for in-situ genotyping the at least one relevant harmful organism,
wherein the sensor arrangement is a lab-on-a-chip device.

2. The method according to claim 1, wherein the method is applied for detecting a DNA-sequence that is indicative of a pesticide resistance, and wherein the sensor arrangement is configured to detect the DNA-sequence that is indicative of the pesticide resistance.

3. The method according to any of claims 1 to 2, wherein the method also comprises outputting, via a human-machine interface functionally coupled to the processing means, information on the type of suitable sensor arrangement to be used.

4. The method according to any of the claims 1 to 3, wherein the method also comprises performing, in-situ using the sensor arrangement connected at a sensor interface, genotyping of the at least one relevant harmful organism from an environmental sample obtained from a crop site, and generating a result signal via the sensor arrangement at the sensor interface; and processing the result signal, using the processing means, for determining whether the one or more of the at least one relevant harmful organism is present in the environmental sample.

5. The method according to claim 4, wherein the method also comprises processing the result signal, using the processing means, for also determining whether the at least one benign organism is present in the environmental sample.

6. The method according to any of the claims 4 to 5, wherein the method also comprises: generating a site result data, using the processing means, by combining the processed result signal and the location data of the crop site where the environmental sample was collected; storing, in at least one database, the site result data; combining data from a plurality of the site result data to obtain a field map representative of the territorial spread of any of the one or more relevant organisms.

7. The method according to claim 6, wherein the method also comprises determining, by analyzing the field map via the processing means, at least one site where another measurement or detection is required.

8. The method according to any of the claims 4 to 7, wherein the method also comprises: determining using the processing means, in response to any of the crop data, the region data, and the selected sensor arrangement; a sampling method for collecting the environmental sample.

9. The method according to any of the claims 4 to 8, wherein the method also comprises: determining, in response to the result signal or the processed result signal, a treatment method for controlling at least one of the at least one relevant harmful organism present on the crop site.

10. The method according to any of the claims 6 to 9, wherein the method further comprises: determining, by analyzing the field map via the processing means, a treatment method for controlling at least one or more harmful organisms present on the crop site.

11. The method according to claim 10, comprising the step of providing, using the processing means, the information on the treatment method as a recommendation to the user.

12. The method according to any of claims 10 or 11, wherein the method also comprises: performing a treatment in accordance to the treatment method to control the at least one of the at least one relevant harmful organism detected on the crop site.

13. An electronic device configured to perform the steps of any of the claims 1 to 12, wherein the electronic device comprises the processing means.

14. A computer program comprising instructions which, when the program is executed by the processing means of an electronic device, cause the electronic device to carry out the method steps of any of the claims 1 to 12.

## Patentansprüche

1. Computerimplementiertes Verfahren, Folgendes umfassend:
- Empfangen, an einem Verarbeitungsmittel, von Erntegutdaten, die für die Art eines Ernteguts bezeichnend sind;
- Empfangen, an dem Verarbeitungsmittel, von Regionsdaten, die für mindestens einen Schadorganismus bezeichnend sind, der an oder um den Ort des Ernteguts herum vorhanden sein kann;
- Auswählen einer Sensoranordnung, die zum selektiven Genotypisieren mindestens eines relevanten Schadorganismus geeignet ist, unter Verwendung des Verarbeitungsmittels aus einer Datenbank; wobei der mindestens eine relevante Schadorganismus unter dem mindestens einen Schadorganismus ist und wobei die Auswahl als Reaktion auf die Erntegutdaten und die Regionsdaten durchgeführt wird,
wobei die Sensoranordnung zum in-situ-Genotypisieren des mindestens einen relevanten Schadorganismus geeignet ist,
wobei die Sensoranordnung eine Lab-on-a-Chip-Vorrichtung ist.

2. Verfahren nach Anspruch 1, wobei das Verfahren zum Detektieren einer DNS-Sequenz angewendet wird, die für eine Pestizidresistenz bezeichnend ist, und wobei die Sensoranordnung eingerichtet ist, um die DNS-Sequenz zu detektieren, die für die Pestizidresistenz bezeichnend ist.

3. Verfahren nach einem der Ansprüche 1 bis 2, wobei das Verfahren auch Ausgeben von Informationen über den Typ einer geeigneten Sensoranordnung, die verwendet werden soll, über eine Mensch-Maschine-Schnittstelle umfasst, die funktional mit dem Verarbeitungsmittel gekoppelt ist.

4. Verfahren nach einem der Ansprüche 1 bis 3, wobei das Verfahren auch Durchführen von Genotypisieren des mindestens einen relevanten Schadorganismus aus einer Umweltprobe, die von einem Erntegutstandort erlangt wurde, in situ unter Verwendung der Sensoranordnung umfasst, die mit einer Sensorschnittstelle verbunden ist, und Erzeugen eines Ergebnissignals über die Sensoranordnung an der Sensorschnittstelle umfasst; und Verarbeiten des Ergebnissignals unter Verwendung des Verarbeitungsmittels zum Bestimmen umfasst, ob der eine oder die mehreren des mindestens einen relevanten Schadorganismus in der Umweltprobe vorhanden sind.

5. Verfahren nach Anspruch 4, wobei das Verfahren auch Verarbeiten des Ergebnissignals unter Verwendung des Verarbeitungsmittels umfasst, um auch zu bestimmen, ob der mindestens eine gutartige Organismus in der Umweltprobe vorhanden ist.

6. Verfahren nach einem der Ansprüche 4 bis 5, wobei das Verfahren auch Folgendes umfasst: Erzeugen von Standortergebnisdaten unter Verwendung des Verarbeitungsmittels durch Kombinieren des verarbeiteten Ergebnissignals und der Ortsdaten des Erntegutstandorts, an dem die Umweltprobe erfasst wurde; Speichern der Standortergebnisdaten in mindestens einer Datenbank; Kombinieren von Daten aus einer Vielzahl der Standortergebnisdaten, um eine Feldkarte zu erlangen, die für die territoriale Ausbreitung eines beliebigen des einen oder der mehreren relevanten Organismen repräsentativ ist.

7. Verfahren nach Anspruch 6, wobei das Verfahren auch Bestimmen durch Analysieren der Feldkarte über das Verarbeitungsmittel von mindestens einem Standort umfasst, an dem eine weitere Messung oder Detektion erforderlich ist.

8. Verfahren nach einem der Ansprüche 4 bis 7, wobei das Verfahren auch Folgendes umfasst: Bestimmen unter Verwendung des Verarbeitungsmittels als Reaktion auf beliebige der Erntegutdaten, der Regionsdaten und der ausgewählten Sensoranordnung eines Probenahmeverfahren zum Erfassen der Umweltprobe.

9. Verfahren nach einem der Ansprüche 4 bis 8, wobei das Verfahren auch Folgendes umfasst: Bestimmen eines Behandlungsverfahrens zum Beherrschen von mindestens einem des mindestens einen relevanten Schadorganismus, der an dem Erntegutstandort vorhanden ist, als Reaktion auf das Ergebnissignal oder das verarbeitete Ergebnissignal.

10. Verfahren nach einem der Ansprüche 6 bis 9, wobei das Verfahren weiterhin Folgendes umfasst: Bestimmen eines Behandlungsverfahrens zum Beherrschen von mindestens einem oder mehreren Schadorganismen, die an dem Erntegutstandort vorhanden sind, durch Analysieren der Feldkarte über das Verarbeitungsmittel.

11. Verfahren nach Anspruch 10, den Schritt des Bereitstellens, unter Verwendung des Verarbeitungsmittels, der Informationen über das Behandlungsverfahren als eine Empfehlung an den Benutzer umfassend.

12. Verfahren nach einem der Ansprüche 10 oder 11, wobei das Verfahren auch Folgendes umfasst: Durchführen einer Behandlung gemäß dem Behandlungsverfahren zum Beherrschen des mindestens einen des mindestens einen relevanten Schadorganismus, der an dem Erntegutstandort detektiert wurde.

13. Elektronische Vorrichtung, die eingerichtet ist, um die Schritte nach einem der Ansprüche 1 bis 12 durchzuführen, wobei die elektronische Vorrichtung das Verarbeitungsmittel umfasst.

14. Computerprogramm, Befehle umfassend, die, wenn das Programm von dem Verarbeitungsmittel einer elektronischen Vorrichtung ausgeführt wird, bewirken, dass die elektronische Vorrichtung die Verfahrensschritte nach einem der Ansprüche 1 bis 12 ausführt.

## Revendications

1. Procédé mis en œuvre par ordinateur comprenant :
- la réception, au niveau d'un moyen de traitement, de données de culture indiquant le type d'une culture ;
- la réception, au niveau du moyen de traitement, de données de région indiquant au moins un organisme nuisible qui peut être présent à ou autour de l'emplacement de la culture ;
- la sélection, dans une base de données à l'aide du moyen de traitement, d'un agencement de capteur adapté au génotypage sélectif d'au moins un organisme nuisible concerné, l'au moins un organisme nuisible concerné se trouvant parmi l'au moins un organisme nuisible, et la sélection étant réalisée en réponse aux données de culture et aux données de région,
dans lequel l'agencement de capteur est adapté au génotypage in situ de l'au moins un organisme nuisible concerné,
dans lequel l'agencement de capteur est un dispositif de laboratoire sur puce.

2. Procédé selon la revendication 1, le procédé étant appliqué à la détection d'une séquence d'ADN qui indique une résistance à un pesticide, et l'agencement de capteur étant conçu pour détecter la séquence d'ADN qui indique la résistance au pesticide.

3. Procédé selon l'une quelconque des revendications 1 à 2, le procédé comprenant également la délivrance, par le biais d'une interface homme-machine fonctionnellement couplée au moyen de traitement, d'informations sur le type d'agencement de capteur adapté à utiliser.

4. Procédé selon l'une quelconque des revendications 1 à 3, le procédé comprenant également la réalisation, in situ à l'aide de l'agencement de capteur connecté à une interface de capteur, du génotypage de l'au moins un organisme nuisible concerné à partir d'un échantillon environnemental obtenu sur un site de culture, et la génération d'un signal de résultat par le biais de l'agencement de capteur à l'interface de capteur ; et le traitement du signal de résultat, à l'aide du moyen de traitement, pour déterminer si le ou les organismes parmi l'au moins un organisme nuisible concerné sont présents dans l'échantillon environnemental.

5. Procédé selon la revendication 4, le procédé comprenant également le traitement du signal de résultat, à l'aide du moyen de traitement, pour déterminer aussi si l'au moins un organisme inoffensif est présent dans l'échantillon environnemental.

6. Procédé selon l'une quelconque des revendications 4 à 5, le procédé comprenant également : la génération de données de résultat de site, à l'aide du moyen de traitement, par combinaison du signal de résultat traité et des données d'emplacement du site de culture où l'échantillon environnemental a été collecté ; le stockage, dans au moins une base de données, des données de résultat de site ; la combinaison de données parmi une pluralité des données de résultat de site pour obtenir une carte de terrain représentative de la dissémination territoriale du ou des organismes concernés quels qu'ils soient.

7. Procédé selon la revendication 6, le procédé comprenant également la détermination, par analyse de la carte de terrain par le biais du moyen de traitement, d'au moins un site où une autre mesure ou détection est nécessaire.

8. Procédé selon l'une quelconque des revendications 4 à 7, le procédé comprenant également : la détermination, à l'aide du moyen de traitement, en réponse à n'importe quel élément parmi les données de culture, les données de région et l'agencement de capteur sélectionné, d'un procédé d'échantillonnage destiné à collecter l'échantillon environnemental.

9. Procédé selon l'une quelconque des revendications 4 à 8, le procédé comprenant également : la détermination, en réponse au signal de résultat ou au signal de résultat traité, d'un procédé de traitement destiné à contrôler l'organisme ou au moins un des organismes nuisibles concernés présents sur le site de culture.

10. Procédé selon l'une quelconque des revendications 6 à 9, le procédé comprenant également : la détermination, par analyse de la carte de terrain par le biais du moyen de traitement, d'un procédé de traitement destiné à contrôler l'organisme ou au moins un des organismes nuisibles présents sur le site de culture.

11. Procédé selon la revendication 10, comprenant l'étape de fourniture, à l'aide du moyen de traitement, des informations sur le procédé de traitement sous la forme d'une préconisation pour l'utilisateur.

12. Procédé selon l'une quelconque des revendications 10 et 11, le procédé comprenant également : la réalisation d'un traitement conformément au procédé de traitement pour contrôler l'au moins un organisme parmi l'au moins un organisme nuisible concerné détecté sur le site de culture.

13. Dispositif électronique conçu pour réaliser les étapes de l'une quelconque des revendications 1 à 12, le dispositif électronique comprenant le moyen de traitement.

14. Programme informatique comprenant des instructions qui, lors de l'exécution du programme par le moyen de traitement d'un dispositif électronique, conduisent le dispositif électronique à réaliser les étapes de procédé de l'une quelconque des revendications 1 à 12.
